# EUROPÄISCHE PATENTANMELDUNG

(11) **EP 3 222 254 A1**
(43) Veröffentlichungstag der Anmeldung: **27.09.2017**
(21) Anmeldenummer: 17160759.1
(22) Anmeldetag: 14.03.2017
(51) Int. Cl.: A61F 2/52

(54) **BRUSTPROTHESE MIT VERSCHLUSSPFROPFEN FÜR EINFÜLLKANAL**

(30) Priorität: 21.03.2016 DE 102016003448
(71) Anmelder: Amoena Medizin-Orthopädie-Technik GmbH, 83064 Raubling (DE)
(72) Erfinder: Stelter, Nils, 83112 Frasdorf (DE)
(74) Vertreter: Laufhütte, Dieter

(57) **Zusammenfassung**

Die Erfindung betrifft ein Verfahren zur Herstellung einer Brustprothese mit einem schalenförmigen Körper, der einen Folienbeutel (1) mit einer Füllung aufweist, wobei das Verfahren die folgenden Schritte aufweist: Verschweißen und/oder Verkleben von Kunststofffolien (2) unter Aussparung eines Bereichs (6), um einen Folienbeutel mit einem Einfüllkanal zu bilden; Einspritzen einer fließfähigen Füllmasse durch den Einfüllkanal in den Folienbeutel; und Verschweißen und/oder Verkleben der Kunststofffolien im Bereich des Einfüllkanals, um den Folienbeutel zu verschließen; wobei, dass nach dem Einspritzen der Füllmasse und vor Verschließen des Folienbeutels eine fließfähige Dichtmasse eingespritzt wird, die zwischen dem Einfüllkanal und der Füllmasse zu liegen kommt und einen Pfropfen (5) bildet.

## Beschreibung

Die Erfindung betrifft ein Verfahren zur Herstellung einer Brustprothese mit einem schalenförmigen Körper, der einen Folienbeutel mit einer Füllung aufweist. Die Erfindung betrifft ferner eine anhand eines derartigen Verfahrens herstellbare Brustprothese.

Brustprothesen umfassend Folienbeutel mit einer fließfähigen Füllmasse haben das Problem, dass die Füllmasse beim Tragen und bei der Qualitätsprüfung einen erhöhten Druck und somit eine erhöhte mechanische Belastung auf die Schweiß- und/oder Klebenähte des Folienbeutels ausübt. Der Schwachpunkt ist dabei der am Ende des Herstellungsverfahrens verschlossene Einfüllkanal, der diesen Belastungen nicht immer standhält. Denn beim Einbringen der Füllmasse lagern sich oft Rückstände im Einfüllkanal an, die ein Verschweißen während eines thermischen Prozesses wie beispielsweise eines Ofenprozesses erschweren oder nur unzureichend ermöglichen. Anschließendes Verschweißen oder Verkleben kann diese Situation verbessern, aber nicht immer zufriedenstellend und dauerhaft lösen.

Aufgabe der Erfindung ist es, ein Verfahren zur Herstellung einer Brustprothese bereitzustellen, mit dem diese Nachteile überwunden werden.

Vor diesem Hintergrund betrifft die Erfindung ein Verfahren zur Herstellung einer Brustprothese mit einem schalenförmigen Körper, der einen Folienbeutel mit einer Füllung aufweist, wobei das Verfahren die folgenden Schritte aufweist: (a) Verschweißen und/oder Verkleben von Kunststofffolien unter Aussparung eines Bereichs, um einen Folienbeutel mit einem Einfüllkanal zu bilden; (b) Einspritzen einer fließfähigen Füllmasse durch den Einfüllkanal in den Folienbeutel; und (c) Verschweißen und/oder Verkleben der Kunststofffolien im Bereich des Einfüllkanals, um den Folienbeutel zu verschließen. Erfindungsgemäß ist vorgesehen, dass nach dem Einspritzen der Füllmasse und vor Verschließen des Folienbeutels eine fließfähige Dichtmasse eingespritzt wird, die zwischen dem Einfüllkanal und der Füllmasse zu liegen kommt und einen Pfropfen bildet.

Sowohl die Füllmasse als auch die Dichtmasse sind so beschaffen, dass sie in den Folienbeutel eingespritzt werden können. Sie sind also fließfähig und vorzugsweise dickflüssig.

In einer Ausführungsform wird die Dichtmasse so eingespritzt, dass sie den Einfüllkanal innen vollständig gegenüber der Füllmasse abdeckt. In einer Ausführungsform wird die Dichtmasse so eingespritzt, dass sie sich haubenförmig von der Einspritzöffnung und deren Umgebung ausgehend in den Folienbeutel ausdehnt und dabei die Füllmasse verdrängt.

In einer Ausführungsform ist die Dichtmasse so ausgebildet, dass sie zu einem elastischen Dichtmaterial verfestigt werden kann.

In einer Ausführungsform handelt es sich bei der Dichtmasse um ein vernetzbares Silikon und vorzugsweise Flüssigsilikon. In einer anderen Ausführungsform handelt es sich bei der Dichtmasse um einen Schmelzklebstoff, der bei einer erhöhten Einspritztemperatur in flüssigem Zustand eingespritzt wird. In einer wiederum anderen Ausführungsform handelt es sich bei der Dichtmasse um einen chemisch härtenden und silikonfreien Kunststoffkleber handelt. Beispiele umfassen Polymerisationsklebstoffe auf beispielsweise Acrylatbasis, Polykondensationsklebstoffe auf beispielsweise Imid- oder Sulfidbasis und Polyadditionsklebstoffe auf beispielsweise Epoxid- oder Urethanbasis.

In einer Ausführungsform weist das Verfahren ferner den Schritt des Verfestigens und vorzugsweise Vernetzens der Dichtmasse auf. Dabei kann es sich beispielsweise um ein thermisches Vernetzen und/oder um eine Strahlenvernetzung handeln. So wird ein gummielastischer Pfropfen gebildet, der im Bereich des ehemaligen Einfüllkanals ein Drücken der Füllmasse auf die Schweiß- und/oder Klebenaht verhindert.

Ein Teil der Dichtmasse kann während des Verschweißens bzw. Verklebens der Kunststofffolien im Bereich der Einfüllöffnung noch zwischen den Kunststofffolien befinden und als Schweißhilfsmittel bzw. thermisch aktivierter Klebstoff dienen.

In einer Ausführungsform ist auch die Füllmasse so ausgebildet, dass die zu einer gummielastischen Masse verfestigt werden kann. In einer Ausführungsform handelt es sich bei der Füllmasse um Flüssigsilikon.

In einer Ausführungsform ist die Füllmasse so ausgebildet, dass sie nicht oder nicht vollständig verfestigt werden kann. Dies kann zur Bildung strukturviskoser Massen mit Fließgrenze führen. Beispiele umfassen geringvernetzte Silikongele. In dieser Ausführungsform kann sich der Pfropfen als besonders vorteilhaft erweisen, da gerade bei fließfähigen Füllungen der Druck auf die Schweiß- bzw. Klebenaht im Bereich der ehemaligen Einfüllöffnung groß sein kann.

Vor dem eingangs genannten Hintergrund betrifft die Erfindung ferner ein Verfahren zur Herstellung einer Brustprothese mit einem schalenförmigen Körper, der einen Folienbeutel mit einer Füllung aufweist, wobei das Verfahren die folgenden Schritte aufweist: (a) Verschweißen und/oder Verkleben on Kunststofffolien unter Aussparung eines Bereichs, um einen Folienbeutel mit einem Einfüllkanal zu bilden; (b) Einspritzen einer fließfähigen und vernetzbaren Füllmasse durch den Einfüllkanal in den Folienbeutel; und (c) Verschweißen und/oder Verkleben der Kunststofffolien im Bereich des Einfüllkanals, um den Folienbeutel zu verschließen. Erfindungsgemäß ist vorgesehen, dass nach dem Einspritzen der Füllmasse und vor Verschließen des Folienbeutels ein Vernetzer eingespritzt wird, der sich im Bereich des Einfüllkanals mit der Füllmasse vermischt, oder dass ein Vernetzer im Bereich des Einfüllkanals außen auf die Kunststofffolien aufgebracht wird.

Dies setzt voraus, dass als Füllmasse ein vernetzbares Material verwendet wird. Es wird also entweder ein Vernetzter nach dem Füllen und vor dem beispielsweise thermischen Vernetzungsprozess für die Füllmasse über den Füllkanal eingebracht, um ein höheres Vernetzen hin zum Pfropfen zu erreichen, oder ein Vernetzter von außen aufgebracht, um beim thermischen Vorgang wie beispielsweise dem Ofenvorgang durch die Folie diffundieren und ebenfalls einen Pfropfenbildung durch zusätzliche Vernetzung bewirken zu können.

Bei dem Vernetzer handelt es sich um eine Verbindung mit zwei oder mehreren reaktiven Gruppen, die mit korrespondierenden reaktiven Gruppen der Füllmass reagieren kann.

Auch der Vernetzer oder alternativ die eingespritzte Trägerzusammensetzung des Vernetzers ist fließfähig und vorzugsweise dickflüssig.

In einer Ausführungsform weist das Verfahren ferner den Schritt des Vernetzens der Füllmasse auf. Dabei kann es sich beispielsweise um ein thermisches Vernetzen und/oder um eine Strahlenvernetzung handeln. Durch das Vorhandensein des Vernetzers nahe der einspritzstelle ist der Vernetzungsgrad dort höher und es bildet sich so ein gummielastischer Pfropfen, der im Bereich des ehemaligen Einfüllkanals ein Drücken der Füllmasse auf die Schweiß- bzw. Klebenaht verhindert.

Das Schweißen der Kunststofffolien kann beispielsweise ein thermisches Schweißen umfassen. Auch andere Verfahren wie beispielsweise Ultraschallschweißen sind denkbar.

Vor dem eingangs genannten Hintergrund betrifft die Erfindung ferner eine Brustprothese mit einem schalenförmigen Körper, der einen Folienbeutel mit einer Füllung aufweist, wobei der Folienbeutel entlang einer Schweiß- und/oder Klebenaht verbundene Kunststofffolien umfasst und wobei sich zwischen der Füllung und einem Teilabschnitt der Schweiß- bzw. Klebenaht ein Pfropfen aus einem Dichtmaterial befindet.

In einer Ausführungsform handelt es sich bei dem Dichtmaterial um ein nicht fließfähiges elastisches Kunststoffmaterial, beispielsweise um Silikonkautschuk. Das nicht fließfähige elastische Kunststoffmaterial kann aber auch silikonfrei sein.

In einer Ausführungsform schließt der Pfropfen haubenförmig an einen Teilabschnitt der Schweiß- bzw. Klebenaht an.

In einer Ausführungsform handelt es sich bei der Füllung um eine strukturviskose Masse mit Fließgrenze, beispielsweise um ein geringvernetztes Silikongel. In einer anderen Ausführungsform handelt es sich bei der Füllung um eine gummielastische Masse, beispielsweise um eine Zweikomponenten-Silikonkautschuk-Masse.

In einer Ausführungsform ist vorgesehen, dass der Folienbeutel durch zwei entlang des gemeinsamen Umfangs verschweißte und/oder verklebte Kunststofffolien gebildet wird.

Der Grundgedanke der vorliegenden Erfindung ist also, den druckanfälligen Einfüllkanal mit einem elastischen Pfropfen zu verschließen, der von dem Füllmaterial ausgehende Drücke abfedert und die an der im Einspritzbereich gebildeten Schweiß- bzw. Klebenaht ankommende Belastungen auf ein Minimum reduziert.

Der Pfropfen resultiert aus einer fließfähigen Masse, die Rückstände der Füllmasse beim Einspritzen aus dem Einfüllkanal drückt und sich dabei haubenförmig ausbildet. Während eines thermischen Prozesses wie beispielsweise eines Ofenprozesses vernetzt bzw. verfestigt sich dieses Material elastisch und bildet eine gute Folienhaftung als Verankerung aus. Somit ist der kritische Einfüllkanal gegen Aufplatzen durch Drücken gut geschützt und verhindert ein Austreten der Füllmasse.

Weitere Einzelheiten und Vorteile der Erfindung werden anhand des in den Figuren dargestellten Ausführungsbeispiels beschrieben. In den Figuren zeigen:

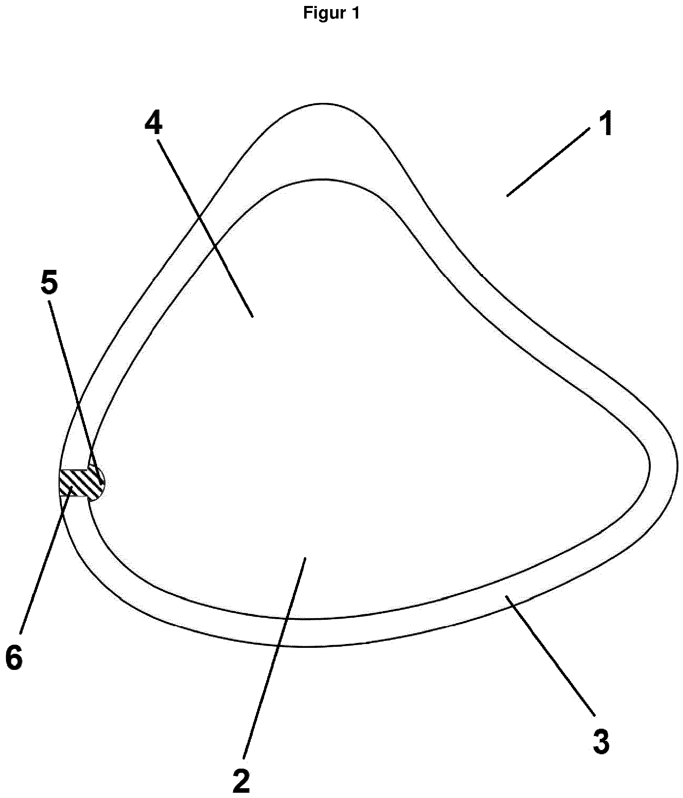

- Figur 1:: eine Draufsicht auf einen Folienbeutel einer erfindungsgemäßen Brustprothese; und

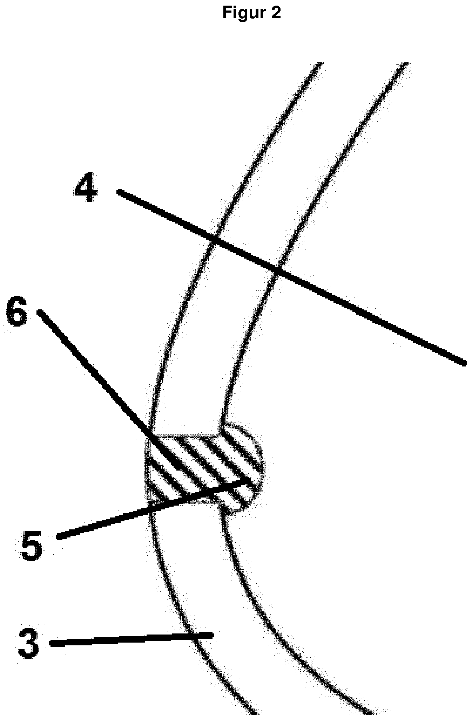

- Figur 2:: eine vergrößerte Darstellung des Bereichs der verschlossenen Einfüllöffnung mit Pfropfen.

Die Figuren zeigen einen schalenförmigen Folienbeutel einer erfindungsgemäßen Brustprothese in Draufsicht. Der Folienbeutel ist allgemein mit dem Bezugszeichen 1 gekennzeichnet. Er bildet neben wenigstens einem weiteren schalenförmigen Folienbeutel, der sich sandwichförmig anschließen kann, sowie gegebenenfalls weiteren Elementen wir textilen Einsätzen eine Brustprothese.

Der Folienbeutel 1 wird durch zwei deckungsgleiche Folienstücke 2 aus einer thermoplastischen Kunststofffolie gebildet, die entlang dem gemeinsamen Umfangsrand verschweißt sind und eine Schweißnaht 3 ausbilden. In der Kammer 4, welche von den Kunststofffolien 2 begrenzt wird, befindet sich ein gering vernetztes Silikongel als Füllmaterial, welches plastisch verformbar ist. Der gezeigte Folienbeutel 1 bildet daher den Teil einer Brustprothese, welche sich plastisch verformen kann und so ermöglicht, dass die Brustprothese der Form des Narbengewebes angepasst werden kann.

In einem kleinen Randbereich der Kammer 4 liegt an der Schweißnaht 3 ein Pfropfen 5 aus vernetztem Silikonkautschuk an. Der Pfropfen erstreckt sich ausgehend von einem Abschnitt 6 der Schweißnaht, an dem sich während des Herstellprozesses des Folienbeutels 1 die Einfüllöffnung befand, haubenförmig in das Innere der Kammer 4. Auch im Bereich 6 der Schweißnaht ist das Material des Pfropfens vorhanden und dient in diesem Bereich als Schweißhilfsmittel bzw. thermisch aktivierter Klebstoff.

Bei der Herstellung des gezeigten Folienbeutels 1 werden zunächst die beiden Kunststofffolien 2 entlang des gemeinsamen Umfangs unter Aussparung des Bereichs 6 zu einem Folienbeutel verschweißt. Im ausgesparten Bereich befindet sich die Einfüllöffnung für die Kammer 4. Anschließend wird eine fließfähige Füllmasse durch den Einfüllkanal in die Kammer 4 gespritzt. Im Anschluss findet ein weiterer Einspritzgvorgang statt, bei dem eine geringe Menge einer vernetzbaren Dichtmasse aus Flüssigsilikon eingespritzt wird, welche später den Pfropfen bildet. Anschließend werden die Kunststofffolien 2 auch im Bereich 6 des Einfüllkanals verschweißt. Das Flüssigsilikon des Pfropfens wird dabei thermisch aktiviert und dient als Schweißhilfsmittel. Bereits während dieses Zuschweißens des Einfüllkanals und auch im Rahmen eines anschließenden Ofenprozesses wird der Pfropfen 5 vulkanisiert, sodass er zu einem gummielastischen Silikonkautschuk aushärtet.

So wird erreicht, dass der Pfropfen den Bereich 6 der Schweißnaht 3, deren herkömmlichen Produkten eine Schwachstelle darstellte, vor Druckeinwirkung beim Tragen und im Rahmen der Qualitätskontrolle schützt und somit die Stabilität und Lebensdauer der Brustprothese erhöht wird.

## Patentansprüche

1. Verfahren zur Herstellung einer Brustprothese mit einem schalenförmigen Körper, der einen Folienbeutel mit einer Füllung aufweist, wobei das Verfahren die folgenden Schritte aufweist:
(a) Verschweißen und/oder Verkleben von Kunststofffolien unter Aussparung eines Bereichs, um einen Folienbeutel mit einem Einfüllkanal zu bilden;
(b) Einspritzen einer fließfähigen Füllmasse durch den Einfüllkanal in den Folienbeutel; und
(c) Verschweißen und/oder Verkleben der Kunststofffolien im Bereich des Einfüllkanals, um den Folienbeutel zu verschließen;
**dadurch gekennzeichnet,**
**dass** nach dem Einspritzen der Füllmasse und vor Verschließen des Folienbeutels eine fließfähige Dichtmasse eingespritzt wird, die zwischen dem Einfüllkanal und der Füllmasse zu liegen kommt und einen Pfropfen bildet.

2. Verfahren nach Anspruch 1, **dadurch gekennzeichnet, dass** die Dichtmasse so ausgebildet ist, dass sie zu einem elastischen Dichtmaterial verfestigt werden kann.

3. Verfahren nach Anspruch 2, **dadurch gekennzeichnet, dass** es sich bei der Dichtmasse um ein vernetzbares Silikon und vorzugsweise Flüssigsilikon handelt.

4. Verfahren nach Anspruch 2, **dadurch gekennzeichnet, dass** es sich bei der Dichtmasse um einen Schmelzklebstoff handelt, der bei einer erhöhten Einspritztemperatur in flüssigem Zustand eingespritzt wird, oder dass es sich bei der Dichtmasse um einen chemisch härtenden und silikonfreien Kunststoffkleber handelt.

5. Verfahren nach einem der vorhergehenden Ansprüche, **dadurch gekennzeichnet, dass** es ferner den Schritt des Verfestigens und vorzugsweise Vernetzens der Dichtmasse aufweist.

6. Verfahren zur Herstellung einer Brustprothese mit einem schalenförmigen Körper, der einen Folienbeutel mit einer Füllung aufweist, wobei das Verfahren die folgenden Schritte aufweist:
(a) Verschweißen und/oder Verkleben von Kunststofffolien unter Aussparung eines Bereichs, um einen Folienbeutel mit einem Einfüllkanal zu bilden;
(b) Einspritzen einer fließfähigen und vernetzbaren Füllmasse durch den Einfüllkanal in den Folienbeutel; und
(c) Verschweißen und/oder Verkleben der Kunststofffolien im Bereich des Einfüllkanals, um den Folienbeutel zu verschließen;
**dadurch gekennzeichnet,**
**dass** nach dem Einspritzen der Füllmasse und vor Verschließen des Folienbeutels ein Vernetzer eingespritzt wird, der sich im Bereich des Einfüllkanals mit der Füllmasse vermischt, oder dass ein Vernetzer im Bereich des Einfüllkanals außen auf die Kunststofffolien aufgebracht wird.

7. Brustprothese mit einem schalenförmigen Körper, der einen Folienbeutel mit einer Füllung aufweist, wobei der Folienbeutel entlang einer Schweiß- und/oder Klebenaht verbundene Kunststofffolien umfasst,
**dadurch gekennzeichnet,**
**dass** sich zwischen der Füllung und einem Teilabschnitt der Schweiß- und/oder Klebenaht ein Pfropfen aus einem Dichtmaterial befindet.

8. Brustprothese nach Anspruch 7, **dadurch gekennzeichnet, dass** es sich bei dem Dichtmaterial um ein nicht fließfähiges elastisches Kunststoffmaterial handelt, beispielsweise um Silikonkautschuk.

9. Brustprothese nach Anspruch 7 oder 8, **dadurch gekennzeichnet, dass** der Pfropfen haubenförmig an einen Teilabschnitt der Schweiß- und/oder Klebenaht anschließt.

10. Brustprothese nach einem der Ansprüche 7 bis 9, **dadurch gekennzeichnet, dass** es sich bei der Füllung um eine strukturviskose Masse mit Fließgrenze, beispielsweise um ein geringvernetztes Silikongel handelt.
